(19) Europäisches Patentamt
European Patent Office
Office européen des brevets

(11) Publication number: 0 293 943 A2

# (12) EUROPEAN PATENT APPLICATION

(21) Application number: 88109005.4

(51) Int. Cl.⁴: C07C 127/22 , A01N 47/34

(22) Date of filing: 06.06.88

(30) Priority: 05.06.87 IT 2080187

(43) Date of publication of application: 07.12.88 Bulletin 88/49

(84) Designated Contracting States: BE DE ES FR GB NL

(71) Applicant: ISTITUTO GUIDO DONEGANI S.p.A.
Via Caduti del Lavoro
I-28100 Novara(IT)

(72) Inventor: Meazza, Giovanni, Dr.
30, via Pastore
I-21047 Saronno Varese(IT)
Inventor: Bettarini, Franco, Dr.
4/B via Cadore
I-28100 Novara(IT)
Inventor: Massardo, Pietro, Dr.
5, via Fra'Bartolomeo
I-20123 Milan(IT)
Inventor: Caprioli, Vincenzo, Dr.
8, via Loriga
I-27028 San Martino Siccomario Pavia(IT)

(74) Representative: Zumstein, Fritz, Dr. et al
Zumstein & Klingseisen Patentanwälte
Bräuhausstrasse 4
D-8000 München 2(DE)

## (54) Benzoyl-ureas having insecticide activity.

(57) There are disclosed compounds having formula:

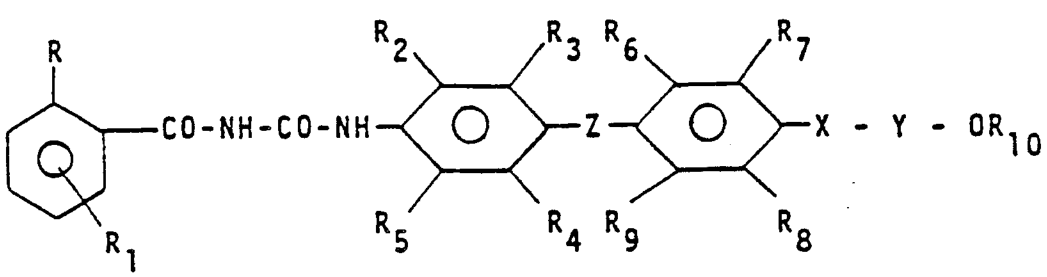

(I)

wherein: R = Cl, F; $R_1$ = H, Cl, F;
$R_2$ and $R_5$, which may be the same or different, are: H, a halogen atom, an alkyl, haloalkyl group;
$R_3$ and $R_4$, which may be the same or different, are: H, a halogen atom, an alkyl, haloalkyl, alkoxy, haloalkoxy group;
Z = O, S;
$R_6$ and $R_9$, which may be the same or different, are: H, a halogen atom, an alkyl, haloalkyl, alkoxy, haloalkoxy group;

EP 0 293 943 A2

$R_7$ and $R_8$, which may be the same or different are: H, a halogen atom, an alkyl, haloalkyl, alkoxy, haloalkoxy group;
X = O, S;
Y = a $C_2$-$C_4$ alkylene, haloethylene or haloethenyl group;
$R_{10}$ = $C_1$-$C_4$ alkyl, $C_1$-$C_4$ haloalkyl, $C_3$-$C_4$ alkenyl, $C_3$-$C_4$ haloalkenyl, $C_3$-$C_4$ cycloalkyl, $C_3$-$C_4$ halocycloalkyl, $C_3$-$C_4$ cycloalkenyl group.

The compounds having formula (I) are endowed with high insecticide activity showing mostly against larvae and eggs of insects.

## BENZOYL-UREAS HAVING INSECTICIDE ACTIVITY

The present invention relates to benzoyl urea derivatives having insecticide activity and more precisely it relates to 1-benzoyl-3-aryl-urea derivatives, particularly active against eggs and larvae of insects which are noxious in agrarian and civil field and to the use of said derivatives.

Moreover the invention relates to the synthesis process of these ureas. Several 1-benzoyl-3-aryl-urea derivatives are known, endowed with insecticide activity.

Among these compounds, the first product put on the commerce was Diflubenzuron, usual name of compound 1-(2,6-difluorobenzoyl)-3-(4-chloro-phenyl)-urea described in U.S. patent 3.933.908 (U.S. Philips Corporation).

Difluorobenzuron, however, as it contains in its molecule the unit of 4-chloro-aniline, is suspected of being a carcinogen [European Chem. News 6 (16), 29 (1978)].

We have now found, and they form an object of the present invention, derivatives of 1-benzoyl-3-aryl-urea having general formula:

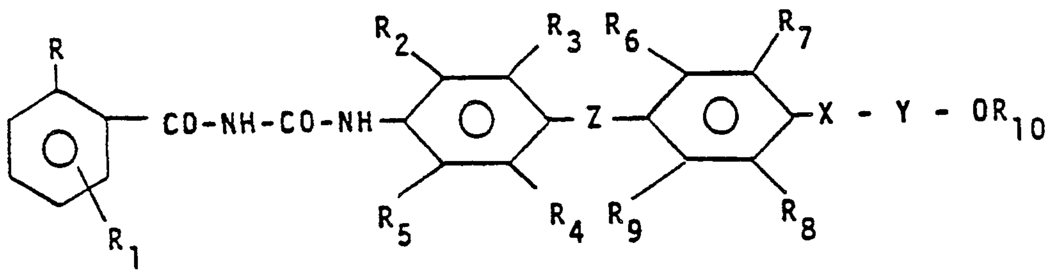
(I)

wherein:

R represents a chlorine or fluorine atom;

$R_1$ represents a hydrogen, chlorine or fluorine atom;

$R_2$ and $R_5$, which may be the same or different, represent a hydrogen, halogen atom, an alkyl group containing from 1 to 4 carbon atoms, a haloalkyl group containing from 1 to 4 carbon atoms;

$R_3$ and $R_4$, which may be the same or different, represent a hydrogen, halogen atom, an alkyl, haloalkyl, alkoxy or halo alkoxy group;

Z represents an oxygen or sulphur atom;

$R_6$ and $R_9$, which may be the same or different, represent a hydrogen, halogen atom, an alkyl, haloalkyl, alkoxy, haloalkoxy group;

$R_7$ and $R_8$, which may be the same or different, represent a hydrogen, halogen atom, an alkyl, haloalkyl, alkoxy, haloalkoxy group;

X represents an oxygen or sulphur atom;

Y represents an alkylene group containing from 2 to 4 carbon atoms, a haloethylene or haloethenyl group or haloethenylene group;

$R_{10}$ represents a $C_1$-$C_4$ alkyl, $C_1$-$C_4$ haloalkyl, $C_3$-$C_4$ alkenyl, $C_3$-$C_4$ haloalkenyl, $C_3$-$C_4$ cycloalkyl, $C_3$-$C_4$ halocycloalkyl or $C_3$-$C_4$ cycloalkenyl group.

In aforesaid definitions, with the term halogen (fluorine, chlorine, bromine and iodine) preferably a fluorine, chlorine or bromine atom is meant, moreover, if it is not indicated otherwise, the alkylic parts of $R_3$, $R_4$, $R_6$, $R_7$, $R_8$ and $R_9$ have to be meant $C_1$-$C_4$ (including $C_1$, $C_2$, $C_3$ and $C_4$ alkylic parts).

The compounds having formula (I) are endowed with insecticide activity and may be used in agrarian, forestal, civil and veterinarian field for fighting insect infestations.

In the description of the preparation of the compounds having formula (I), as set forth hereinafter, symbols R, $R_1$, $R_2$, $R_3$, $R_4$, $R_5$, $R_6$, $R_7$, $R_8$, $R_9$, $R_{10}$, X, Y, Z have the meanings set forth in formula (I), if it is not specified otherwise.

The compounds having formula (I) are obtained by letting react a benzoyl-isocyanate having formula:

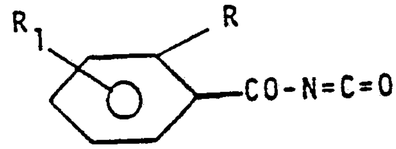

(II)

with an aromatic amine having formula:

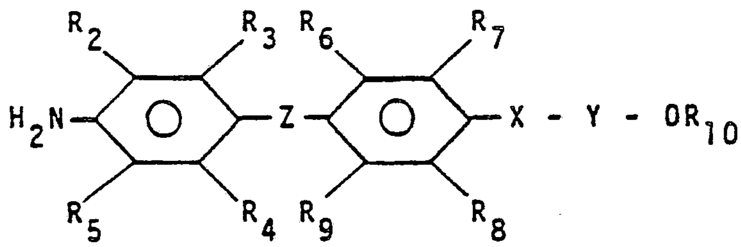

(III)

The reaction does not require the presence of catalysts and is carried out in an inert solvent and at temperatures ranging from 0 °C to the boiling temperature of the mixture.

Aromatic hydrocarbons, chlorinated hydrocarbons, ethers, ketones and acetonitrile are suitable solvents.

The benzoylisocyanates having formula (II) are known compounds and at times they can be found on the market.

The amines having formula (III) can be prepared, according to known methods, for instance: by letting react the sodium or potassium salt of a suitable aminophenoxy phenol (V), in dipolar aprotic solvents or mixtures of such solvents with aromatic hydrocarbons, with ethereal compound:

$CF_2 = CF\text{-}OR_{10}$ (IV)

wherein $R_{10}$ has the meaning given in formula (I), at temperatures ranging from 10 to 40 °C, according to the following equation:

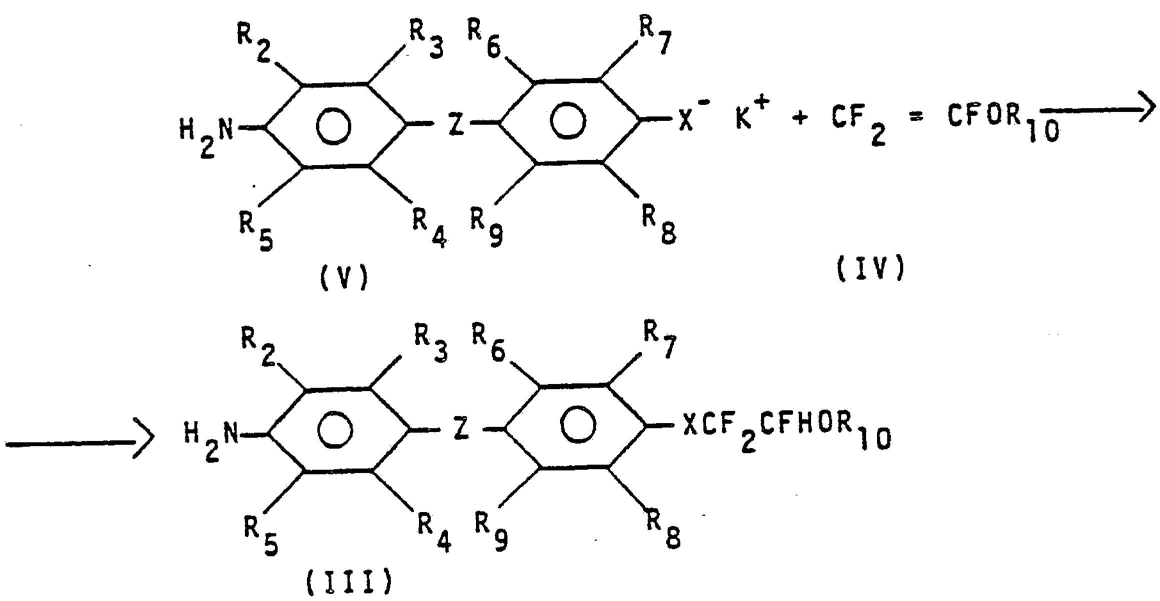

(V) (IV)

(III)

The ethers having formula (IV) can be prepared according to known methods, described for instance in J. Am. Chem. Soc., 82, 5116 (1960) when $R_{10}$ is an alkyl or aryl group and in J. Org. Chem., 48, 242 (1983) when $R_{10}$ is a polyhaloalkyl group.

As it is evident to a person skilled in the art, several alternative procedures are possible for the synthesis of the intermediates and of the products having formula (I).

An alternative procedure for the synthesis of compounds having formula (I) consists, for instance, in letting react a benzamide having formula:

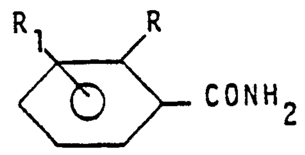

(VI)

with a isocyanate having formula:

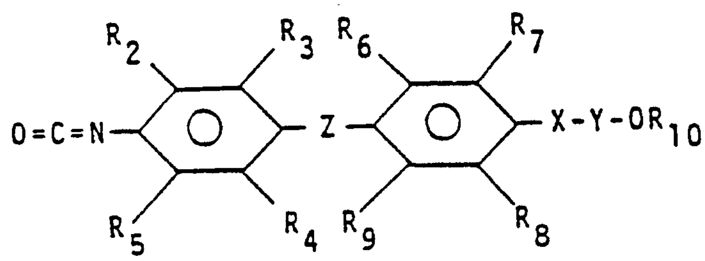

(VII)

Such reaction is carried out under conditions similar to the ones above described for the reaction between the benzoyl isocyanate having formula (II) and the amine having formula (III).

The preparation of the isocyanates having formula (VII) foresees , however, the preparation of the amines having formula (III) and the reaction of said amines with phosgene.

This point of view, together with the fact that the benzoyl-isocyanates having formula (II) are available as the amides of formula (VI) are, leads generally to prefer the synthesis method reported hereinbefore, namely the reaction between the compounds having formula (II) and (III).

As above mentioned, the compounds having formula (I) are endowed with high insecticide activity, showing mostly against larvae and eggs of insects.

Among these insects, the ones belonging to the Lepidoptera, Diptera and Coleoptera orders can be fought particularly by means of the compounds having formula (I).

These orders comprise several species that are important owing to their noxiousness in agrarian, forestal, civil and veterinarian field. Therefore the compounds having formula (I) are fit for different uses, for instance, for protecting agricultural cultivations, from infestations of phytophagous insects, for protecting environments infested by mosquitoes and flies, for protecting breeding-cattle from a few cattle parasites and so on.

Moreover the compounds having formula (I) show a collateral acaricide activity.

For the practical uses the compounds having general formula (I) may be used as such or, more suitably, in the form of compositions containing, in addition to one or more of the compounds of formula (I) as active principle, solid or liquid inert vehicles and optionally other additives as well. According to the customary formulative praxis, the compositions can be in the form of wettable powders, emulsifiable concentrates, dispersable powders, aqueous solutions or dispersions and the like.

The amount of active principle in the compositions ranges within wide limits (1-95% by weight), depending on the type of composition and on the use such a composition is allotted to.

If required by particular situations or in order to extend the action spectrum, other active substances such as, for instance, other insecticides or acaricides, can added to the compositions. The amount of active substance (compound of formula I) to be distributed for the insecticide treatments depends on different factors such as, for instance, the kind of infestation and its degree, the environment wherein the infestation breaks out (agrarian cultivations, basins and water-courses, organic substrates of different nature, and the like),the kind of composition that has been used, climatic and environmental factors, available application means and so on. In general, amounts of active substance ranging from 0.01 to 1 kg/ha are sufficient for a good disinfestation. The following examples will now better illustrate the invention.

In the proton nuclear magnetic resonance spectra($^1$H-NMR), set forth in examples, use is made of the following abbreviations:

s = singlet;

m = multiplet or unresolved complex signal;

t = triplet;

b = (broad) = broadened signal.

EXAMPLE 1

Preparation of N-2,6-difluorobenzoyl-N′-3-chloro-4-[4-(1,1,2,-trifluoro-2-(trifluoromethoxy)ethoxy)phenoxy] phenylurea (compound No 1).

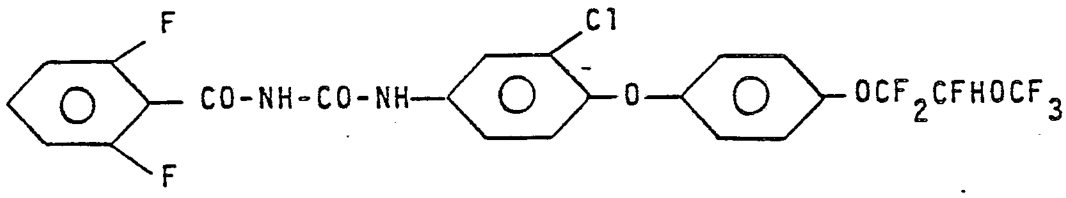

1.0 g of 3-chloro-4-[-4-(1,1,2,-trifluoro-2-(trifluoromethoxy)ethoxy)phenoxy] aniline (prepared as described in subsequent example 3) dissolved in 15 ml of anhydrous ethyl ether, was introduced, in a nitrogen atmosphere, into a 50 ml flask equipped with a cooler, dropping funnel, thermometer and magnetic stirrer. Then 0.46 g of 2,6-difluorobenzoylisocyanate dissolved in 4.5 ml of anhydrous chlorobenzene, were dripped there at room temperature. The mixture was heated at 50° C over 60 minutes, cooled and filtered. The filtrate was concentrated at reduced pressure and the product was separated by filtration.

One obtained 0.77 g of benzoylurea having a melting point of 133-135° C.

EXAMPLE 2

By starting from the anilines described in subsequent example 3 and by operating under conditions similar to the ones described in example 1, the following compounds were obtained, by using 2,6-difluorobenzoylisocyanate:

- Compound No 2

N-2,6-difluorobenzoyl-N′-4-[4-(1,1,2,-trifluoro-2-(trifluoro-methoxy)ethoxy)phenoxy]phenylurea:

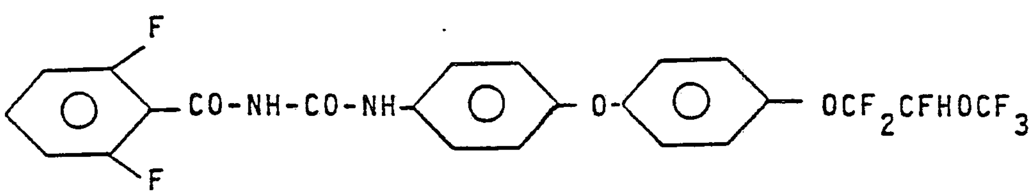

m.p. 153-158° C.

- Compound No 3

N-2,6-difluorobenzoyl-N′-2-fluoro-4-[4-(1,1,2-trifluoro-2-(trifluoromethoxy)ethoxy)phenoxy]phenylurea.

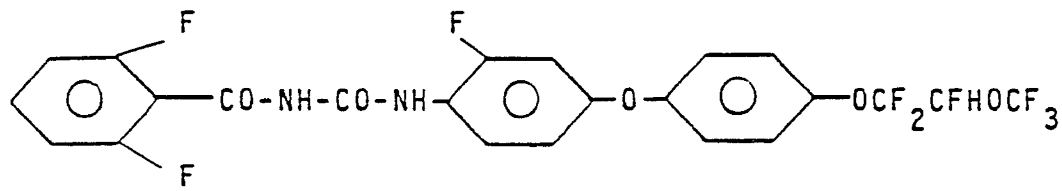

m.p. 118-120° C.

- Compound. No 4

N-2,6-difluorobenzoyl-N′-4-[3-chloro-4-(1,1,2,-trifluoro-2-(trifluoromethoxy)ethoxy)phenoxy]phenylurea:

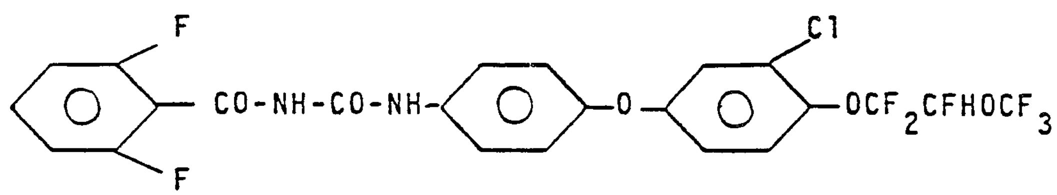

m.p. 139-141° C.

- Compound No 5

N-2,6-difluorobenzoyl-N′-4-[2-chloro-4-(1,1,2-(trifluoro)-2-(trifluoromethoxy)-ethoxy)phenoxy]phenylurea:

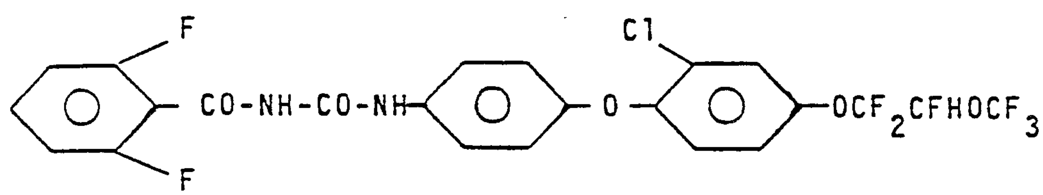

m.p. 120-122° C.

While using 2-chlorobenzoyl isocyanate the following compounds were prepared:

- Compound No 6

N-2-chlorobenzoyl-N′-3-chloro-4-[4-(1,1,2,-trifluoro-2-(trifluoromethoxy)ethoxy)phenoxy]phenylurea:

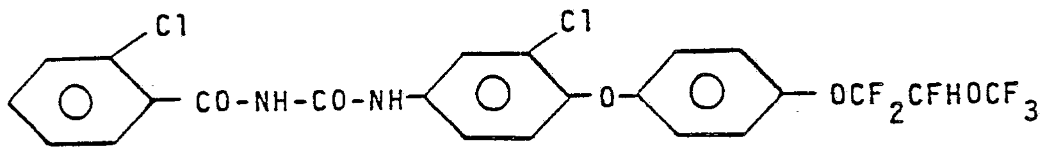

m.p. 130-132° C.

- Compound No 7:

N-2-chlorobenzoyl-N′-4-[4-(1,1,2,-trifluoro-2-(trifluoromethoxy)ethoxy)phenoxy]phenylurea:

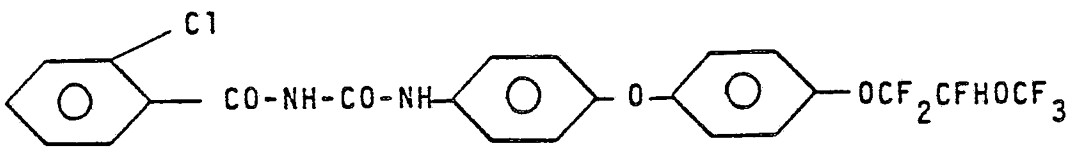

m.p. 150-153° C.

- Compound No 8:

N-2-chlorobenzoyl-N′-2-fluoro-4-[4-(1,1,2,-trifluoro-2-(trifluoromethoxy)ethoxy)phenoxy]phenylurea:

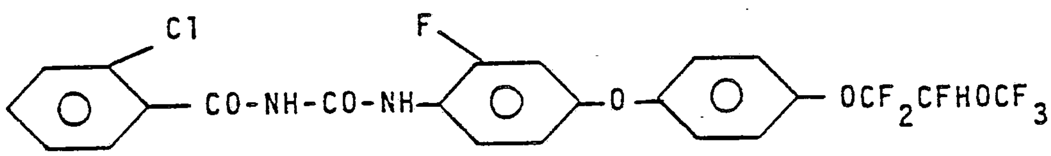

m.p. 103-104° C.

- Compound No 9:

N-2-chlorobenzoyl-N′-4-[3-chloro-4-(1,1,2,-trifluoro-2-(trifluoromethoxy)ethoxy)phenoxy]phenylurea:

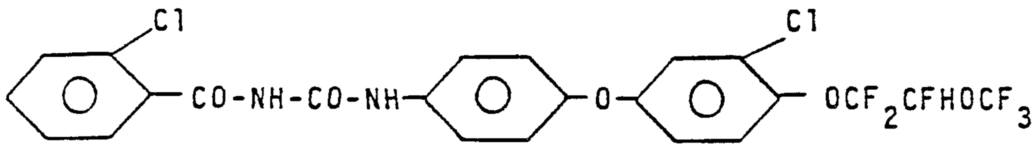

m.p. 119-122° C.

- Compound No 10:

N-2-chlorobenzoyl-N′-4-[2-chloro-4-(1,1,2,-trifluoro-2-(trifluoromethoxy)ethoxy)phenoxy]phenylurea:

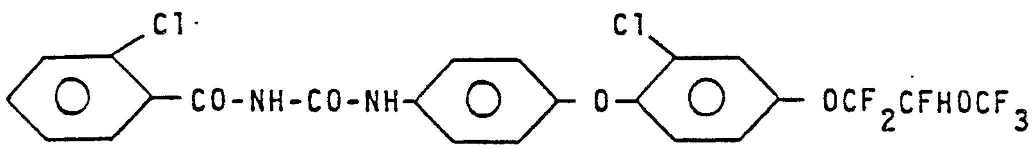

m.p. 108-110° C.

EXAMPLE 3

Preparation of intermediate anilines.

6.93 g of 3-chloro-4-(4-hydroxy-phenoxy)aniline, 6.1 ml of dimethyl sulfoxide, 24.4 ml of toluene and 1.65 g of ground potassium hydroxide were loaded into a two neck flask equipped with a thermometer, magnetic stirrer and gas feeding device.

One produced the vacuum in the flask, and one linked said flask with a reservoir containing 4.6 g of perfluoromethylperfluorovinylether.

The reaction mixture was stirred over 60 minutes at room temperature. One poured into water, one extracted with ethyl ether, one dehydrated and one concentrated under vacuum.

The obtained raw product was purified by chromatography on silica gel column.

One obtained 6.53 g of 3-chloro-4-[4-(1,1,2-trifluoro-2-(trifluoromethoxy)ethoxy)phenoxy]aniline.

$^1$H-NMR ($CDCl_3$): 7.1-6.15 (m, 7.5 H); 5.3 (t, 0.5 H); 3.5 (bs,2H).

By operating under conditions similar to the ones described hereinbefore, 4-[4-(1,1,2,-trifluoro-2-(trifluoromethoxy)ethoxy)phenoxy]aniline was obtained, starting from 4-(4-hydroxy-phenoxy)aniline.

$^1$H-NMR ($CDCl_3$): 7.15-6.15 (m, 8.5 H); 5.35 (t, 0.5 H); 4.0 (bs, 2H).

2-fluoro-4-[4-(1,1,2-trifluoro-2-trifluoromethoxy) ethoxy)-phenoxy]aniline was obtained starting from 2-fluoro-4-(4-hydroxy-phenoxy)aniline.

$^1$H-NMR ($CDCl_3$): 7.1-6.1 (m, 7.5 H); 5.35 (t, 0.5 H); 3.5 (bs, 2H).

4-[3-chloro-4-(1,1,2,-trifluoro-2-(trifluoromethoxy) ethoxy)phenoxy]aniline was obtained, starting from 4-(3-chloro-4-hydroxy-phenoxy)aniline.

$^1$H-NMR ($CDCl_3$): 7.3-6.2 (m, 7.5 H); 5.4 (t, 0.5 H); 3.5 (bs, 2H).

4-[2-chloro-4-(1,1,2,-trifluoro-2-(trifluoromethoxy) etoxy)phenoxy]aniline was obtained starting from 4-(2-chloro-4-hydroxy-phenoxy)aniline.

$^1$H-NMR ($CDCl_3$): 7.2-6.2 (m, 7.5 H); 5.4 (t, 0.5 H); 3.5 (bs, 2H).

## EXAMPLE 4

Determination of the insecticide activity.

### Test 1

Immediate residual activity on larvae of Spodoptera littoralis (Lepidoptera).

Tobacco leaves were sprayed mechanically with a water-acetone solution (10% by volume of acetone) containing a surfactant and the product being tested.

After complete removal of the solvents, the leaves were infested with second age larvae of Lepidoptera.

The infested leaves were kept in a suitably conditioned place during the time required for the test.

Tobacco leaves, treated only with a water-acetone solution (10% of acetone) containing the surfactant, to be used as comparison (comparison test), were infested and kept likewise.

Ten days after the infestation and after having renewed at least once the treated substrate, the reckoning was carried out of the dead larvae in comparison with the comparison test.

### Test 2

Activity on larvae of Aedes aegypti (Diptera).

Tap water (297 ml) was mixed with an acetone solution (3 ml) containing the product being tested in a suitable concentration.

25 larvae of Diptera, being 4 days of age and fed suitably, were introduced into the obtained solution. Other larvaes were introduced, as comparison test, into a water-acetone solution (acetone 3 ml, tap water 297 ml), containing no active product.

Every 2-3 days a note was made of dead larvae and pupae and of adult insects emerged from the cocoon normally, till the emergence from the cocoon of the insects, in the comparison test, was over.

The activity of the product being tested was expressed as per cent ratio of dead individuals compared with the total number of treated individuals.

The insecticide activity in aforesaid tests was expressed according to the following scale of valves:

5 = complete activity (98-100% of mortality)
4 = high activity (80-97% of mortality)
3 = fairly good activity (60-79% of mortality)
2 = sufficient activity (40-59% of mortality)
1 = scanty activity (20-39% of mortality)
0 = negligible or no activity (0-19% of mortality)

The data of insecticide activity at the indicated doses, expressed according to the above reported scale of values, are set forth in following Table 1.

TABLE 1

| Insecticide activity | | | |
|---|---|---|---|
| Compound No | Test 1 | | Test 2 |
| | dose | | dose |
| | 50 ppm | 10 ppm | 0.2 ppm |
| 1 | 5 | 5 | 5 |
| 2 | 5 | 5 | 5 |
| 3 | 5 | 5 | 5 |
| 4 | 5 | 5 | 5 |
| 5 | 5 | 5 | 5 |
| 6 | 5 | 5 | 5 |
| 7 | 5 | 5 | 5 |
| 8 | 5 | 5 | 5 |
| 9 | 5 | 5 | 5 |
| 10 | 5 | 5 | 5 |

## Claims

1. Compounds having formula:

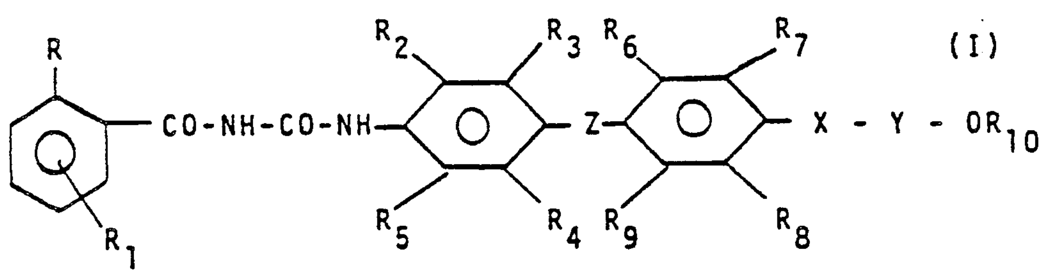

(I)

wherein:
R represents a chlorine or fluorine atom;
$R_1$ represents a hydrogen, chlorine or fluorine atom;
$R_2$ and $R_5$, which may be the same or different, represent a hydrogen, halogen atom, an alkyl group containing from 1 to 4 carbon atoms, a haloalkyl group containing from 1 to 4 carbon atoms;
$R_3$ and $R_4$, which may be the same or different, represent a hydrogen, halogen atom, an alkyl, haloalkyl, alkoxy or haloalkoxy group;
Z represents an oxygen or sulphur atom;
$R_6$ and $R_9$, which may be the same or different, represent a hydrogen, halogen atom, an alkyl, haloalkyl, alkoxy or haloalkoxy group;
$R_7$ and $R_8$, which may be the same or different, represent a hydrogen, halogen atom, an alkyl, haloalkyl, alkoxy or halo alkoxy group;
X represents an oxygen or sulphur atom;
Y represents an alkylene group containing from 2 to 4 carbon atoms, a haloethylene or haloethenyl group;
$R_{10}$ represents a $C_1$-$C_4$ alkyl, $C_1$-$C_4$ haloalkyl, $C_3$-$C_4$ alkenyl, $C_3$-$C_4$ haloalkenyl, $C_3$-$C_4$ cycloalkyl, $C_3$-$C_4$ halocycloalkyl or $C_3$-$C_4$ cycloalkenyl group..

2. A compound according to claim 1, namely:
N-2,6-difluorobenzoyl-N′-3-chloro-4-[4-(1,1,2-trifluoro-2-(trifluoromethoxy)ethoxy)phenoxy]phenylurea.

3. A compound, according to claim 1, namely:
N-2,6-difluorobenzoyl-N′-4-[4-(1,1,2-trifluoro-2-(trifluoromethoxy)ethoxy)phenoxy]phenylurea.

4. A compound according to claim 1, namely:
N-2,6-difluorobenzoyl-N′-3-fluoro-4-[4-(1,1,2-trifluoro-2-(trifluoromethoxy)ethoxy)phenoxy]phenylurea.

5. A compound according to claim 1, namely:
N-2,6-difluorobenzoyl-N′-4-[3-chloro-4-(1,1,2-trifluoro-2-(trifluoromethoxy)ethoxy)phenoxy]phenylurea.

6. A compound according to claim 1, namely:
N-2,6-difluorobenzoyl-N′-4-[2-chloro-4-(1,1,2-(trifluoro)-2-(trifluoromethoxy)ethoxy)phenoxy]phenylurea.

7. A compound according to claim 1, namely:
N-2-chlorobenzoyl-N′-3-chloro-4-[4-(1,1,2-trifluoro-2-tri fluoromethoxy)ethoxy)phenoxy]phenylurea.

8. A compound according to claim 1, namely:
N-2-chlorobenzoyl-N′-4-[4-(1,1,2,-trifluoro-2-(trifluoromethoxy)ethoxy)phenoxy]phenylurea.

9. A compound according to claim 1, namely:
N-2-chlorobenzoyl-N′-2-fluoro-4-[4-(1,1,2,-trifluoro-2-(trifluoromethoxy)ethoxy)phenoxy]phenylurea.

10. A compound according to claim 1, namely:
N-2- chlorobenzoyl-N′-4-[3-chloro-4-(1,1,2,-trifluoro-2-(trifluoromethoxy)ethoxy)phenoxy]phenylurea.

11. A compound according to claim 1, namely:
N-2-chlorobenzoyl-N′-4-[2-chloro-4-(1,1,2,-trifluoro-2-(trifluoromethoxy)ethoxy)phenoxy]phenylurea.

12. A process, for preparing the compounds according to claim 1, characterized in that a benzoyl-isocyanate having formula:

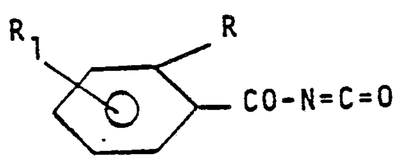

(II)

is let react with an aromatic amine having formula:

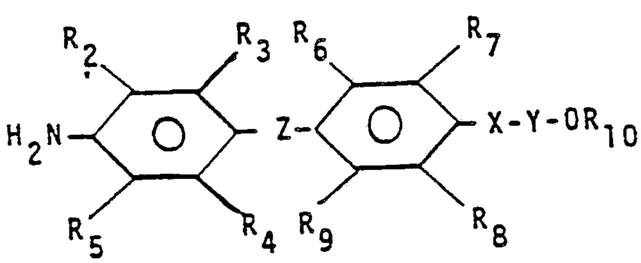

(III)

in which formulas symbols R, $R_1$, $R_2$, $R_3$, $R_4$, $R_5$, $R_6$, $R_7$, $R_8$, $R_9$, $R_{10}$ X, Y and Z have the meanings as

defined in claim 1 and the reaction is carried out in an inert organic solvent, at temperatures ranging from 0 °C to the boiling temperature of the reaction mixture.

13. A method of fighting infestations of noxious insects consisting in distributing in the infestation area an effective amount of one or more of the compounds according to claim 1, as such or in the form of a suitable composition.

14. Insecticide compositions containing as active ingredient, one or more of the compounds according to claim 1, together with liquid or solid inert vehicles and optionally other additives and/or other active substances.